# EUROPÄISCHE PATENTSCHRIFT

(11) **EP 2 229 357 B1**
(45) Veröffentlichungstag und Bekanntmachung des Hinweises auf die Patenterteilung: **05.06.2013**
(21) Anmeldenummer: 08856062.8
(22) Anmeldetag: 26.11.2008
(51) Int. Cl.: C07D 207/267

(54) **Verfahren zur Herstellung von N-Vinylpyrrolidon aus lactonfreiem Pyrrolidon**
Method for producing N-vinyl pyrrolidone from lactone-free pyrrolidone
Procédé de preparation de N-vinylpyrrolidone à partir de pyrrolidone exempte de lactone

(30) Priorität: 07.12.2007 EP 07122636
(43) Veröffentlichungstag der Anmeldung: 22.09.2010
(73) Patentinhaber: BASF SE, 67056 Ludwigshafen (DE)
(72) Erfinder: STAFFEL, Wolfgang, 67165 Waldsee (DE); KÄSHAMMER, Stefan, 67105 Schifferstadt (DE); KESSINGER, Roland, 69469 Weinheim (DE); VOGELSANG, Regina, 67063 Ludwigshafen (DE); PAUL, Axel, 68623 Lampertheim (DE); TUTTELBERG, Lembit, 68305 Mannheim (DE)
(86) Internationale Anmeldenummer: PCT/EP2008/066251
(87) Internationale Veröffentlichungsnummer: WO 2009/071479

(56) Entgegenhaltungen:
- WO-A1-03/022811
- DE-A1- 1 795 007
- US-A- 4 873 336
- "2-Pyrrolidone" In: ELVERS, B. ET AL.: "Ullmann's Encyclopedia of Industrial Chemistry, 5th Edition", 1993, VCH Verlagsgesellschaft, Weinheim; DE ISBN: 3-527-20122-X vol. A 22, pages 458-463, * page 459, column 2, paragraph 4 - page 460, column 1, paragraph 3 *

## Beschreibung

Die Erfindung betrifft ein Verfahren zur Herstellung von N-Vinylpyrrolidon durch Umsetzung von 2-Pyrrolidon mit Acetylen, welches dadurch gekennzeichnet ist, dass das als Ausgangsstoff eingesetzte 2-Pyrrolidon (im nachfolgenden Ausgangs-2-Pyrrolidon genannt) weniger als 1 Gew.-Teile y - Butyrolacton auf 100 Gew.-Teile 2- Pyrrolidon enthält.

N-Vinylpyrrolidon wird technisch durch Vinylierung von 2- Pyrrolidon mit Acetylen hergestellt. 2- Pyrrolidon (γ -Butyrolactam) wiederum ist durch Umsetzung von Butyrolacton mit Ammoniak erhältlich, wie z. B. in DE-A 1 795 007 beschrieben ist. Das bei der Herstellung von N-Vinylpyrrolidon verwendete 2-Pyrrolidon enthält daher im Allgemeinen noch Restmengen an γ -Butyrolacton.

Aufgabe der vorliegenden Erfindung war ein Verfahren zur Herstellung von N-Vinylpyrrolidon mit hohen Raum-Zeit-Umsätzen und möglichst hohen Ausbeuten an N-Vinylpyrrolidon.

Demgemäß wurde das eingangs definierte Verfahren gefunden.

Ausgangsstoffe für das Verfahren sind 2-Pyrrolidon und Acetylen.

Das beim erfindungsgemäßen Verfahren eingesetzte 2-Pyrrolidon wird im nachfolgenden als Ausgangs-2-Pyrrolidon bezeichnet.

2- Pyrrolidon wird auch als γ -Butyrolactam bezeichnet und ist die bekannte Verbindung der Formel

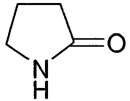

Als Ausgangs-2-Pyrrolidon wird erfindungsgemäß ein Ausgangsstoff verwendet, der weniger als 1 Gew.-Teile, bevorzugt weniger als 0,5 Gew.-Teile und besonders bevorzugt weniger als 0,3, insbesondere weniger als 0,15 und ganz besonders bevorzugt weniger als 0,1 Gew.-Teil Gew.-Teil y - Butyrolacton der Formel 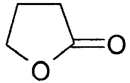 auf 100 Gew.-Teile 2- Pyrrolidon enthält.

γ - Butyrolacton kann aus 2-Pyrrolidon nachträglich im gewünschten Ausmaß entfernt werden. Alternativ kann auch bereits die Herstellung von 2-Pyrrolidon so erfolgen, dass das erhaltene 2-Pyrrolidon nicht mehr als die oben angegebenen Mengen an γ - Butyrolacton enthält. 2-Pyrrolidone mit den oben angegebenen maximalen Gehalten an γ - Butyrolacton ist im Markt erhältlich.

Geeignete Ausgangs-2-Pyrrolidone können andere Verunreinigungen oder Nebenprodukte enthalten, vorzugsweise enthalten sie diese, wenn überhaupt, nur in geringen Mengen.

Insbesondere kann geeignetes Ausgangs-2-Pyrrolidon auf 100 Gew.-Teile 2-Pyrrolidon gegebenenfalls folgende weiteren Bestandteile aufweisen:
0 bis 2 Gew.-Teile, insbesondere 0 bis 1 Gew.-Teil besonders bevorzugt 0 bis
0,1 Gew.-Teil Wasser und
0 bis 2 Gew.-Teile, insbesondere 0 bis 1 Gew.-Teil besonders bevorzugt 0 bis
0,1 Gew.-Teil Methylpyrrolidon.

Das Ausgangs-2-pyrrolidon besteht vorzugsweise zu mehr als 97 Gew.-%, insbesondere zu mehr als 98 Gew.-%, besonders bevorzugt zu mehr als 98,5 Gew.-% und ganz besonders bevorzugt zu mehr als 99 Gew.-%, insbesondere zu mehr als 99,5 Gew.-% bzw zu mehr als 99,7 Gew.-% aus 2-Pyrrolidon.

Auch das eingesetzte Acetylen kann gegebenenfalls Nebenprodukte und Verunreinigungen enthalten. Insbesondere kann geeignetes Acetylen gegebenenfalls auf 100 Gew.-Teile Acetylen noch bis zu 2 Gew.-Teilen, insbesondere bis zu 1 Gew.-Teil Propin enthalten.

Die Umsetzung des Ausgangs-2 Pyrrolidons mit Acetylen erfolgt vorzugsweise in Gegenwart eines Katalysators.

Als Katalysator haben sich insbesondere Alkalimetall -pyrrolidate bewährt.

Dazu wird Ausgangs-2-Pyrrolidon vorzugsweise zunächst mit einem Alkalihydroxid oder Alkalialkoholat umgesetzt. Es kann sich z. B. um ein Lithium-, Natrium- oder Kaliumhydroxid handeln; besonders bevorzugt ist Kaliumhydroxid.

Das Alkalihydroxid wird vorzugsweise in Form einer wässrigen Lösung verwendet. Der Gehalt an Alkalihydroxid kann z. B. 5 bis 90 Gew.-%, bezogen auf die Lösung betragen; insbesondere beträgt er 30 bis 60 Gew.-%, besonders bevorzugt 45 bis 55 Gew.-%.

Die Umsetzung mit dem Alkalihydroxid erfolgt vorzugsweise bei Temperaturen von 50 bis 250°C und 1 mbar bis 1 bar, insbesondere bei 20 bis 250°C. Die Temperatur am Kolonnenkopf beträgt vorzugsweise 20 bis 100, insbesondere 25 bis 60°C; die Temperatur im Kolonnensumpf beträgt vorzugsweise 100 bis 250°C, insbesondere 120 bis 200°C.

Diese Umsetzung wird vorzugsweise semi-kontinuierlich oder kontinuierliche betrieben. Besonders bevorzugt wird sie kontinuierlich betrieben.

Vorzugsweise erfolgt die Umsetzung in einer Kolonne, besonders bevorzugt in einer Füllkörperkolonne oder Packungskolonne, welche bei den vorstehenden Temperaturen und Drucken betrieben wird. Sie wird vorzugsweise kontinuierlich betrieben.

Besonders bevorzugt sind Kolonnen, die sowohl Füllkörper als auch Packungen enthalten, z. B. im unteren teil Füllkörperschüttungen enthalten und im oberen Teil mit Packungselementen (z.B. eingebaute Stahlbleche) aufweisen.

Die Kolonne hat vorzugsweise mindestens zwei, besonders bevorzugt mindestens 3 theoretische Böden. Sie kann z. B. 2 bis 100, insbesondere 3 bis 20 theoretische Böden haben.

Das Alkalihydroxid und 2-Pyrrolidon werden vorzugsweise im oberen Drittel, besonders bevorzugt im oberen Viertel der Kolonne zugegeben.

Die mittlere Verweilzeit des Alkalihydroxids und des Ausgangs-2-pyrrolidons in der Reaktionszone, bzw. Kolonne, ist kleiner 6 Minuten, insbesondere kleiner 5 Minuten, besonders bevorzugt beträgt sie 50 bis 200 Sekunden.

Bei der Umsetzung bildet sich das entsprechende Alkalisalz, d.h. das Alkali-pyrrolidat, vorzugsweise Kaliumpyrrolidat.

Die Menge an Alkalihydroxid wird vorzugsweise so gewählt, dass 0,25 bis 25 Gew.-%, vorzugsweise 5 bis 20 Gew.-% des 2-Pyrrolidons als Pyrrolidat, bzw. Kaliumpyrrolidat vorliegen.

Das Produkt der Umsetzung kann im unteren Teil der Kolonne oder am Kolonnensumpf kontinuierlich abgezogen werden. Anschließend erfolgt dann vorzugsweise in einem separaten Reaktionsgefäß die Umsetzung mit Acetylen.

Das Alkalisalz katalysiert die anschließende Umsetzung mit Acetylen (Vinylierung). Der Austrag aus der vorstehenden Kolonne kann mit weiterem Ausgangs-2-Pyrrolidon gemischt werden.

Das für die Vinylierung verwendete 2-Pyrrolidon liegt dann vorzugsweise zu 0,25 bis 10 Gew.-% , insbesondere 1,5 bis 6 Gew.-% als Pyrrolidat vor.

Die anschließende Vinylierung mit Acetylen kann ebenfalls diskontinuierlich, semikontinuierlich oder kontinuierlich erfolgen.

Die Vinylierung erfolgt vorzugsweise kontinuierlich.

Die Umsetzung mit Acetylen wird vorzugsweise bei Temperaturen von 120 bis 220, besonders bevorzugt bei 140 bis 170°C und vorzugsweise bei Drucken von 1,0 bis 25 und besonders bevorzugt bei 10 bis 20 bar durchgeführt.

Durch das erfindungsgemäße Verfahren ist N-Vinylpyrrolidon in hoher Ausbeute und Reinheit erhältlich. Dagegen zeigt die Verwendung von Ausgangs-2-Pyrrolidon, welches mehr als 1 Gew.-Teile γ - Butyrolacton auf 100 Gew.-Teile 2- Pyrrolidon enthält, weitaus schlechtere Ausbeuten. Die Verschlechterung der Ausbeute an Vinylpyrrolidon geht über den bloßen Gehalt an y - Butyrolacton, welches nicht vinyliert wird, hinaus. Die Gegenwart von mehr als 1 Gew.-Teil γ - Butyrolacton beeinträchtigt daher in nicht nahe liegender und unverhältnismäßiger Weise die Ausbeute an Vinylpyrrolidon.

### Beispiele

### Beispiel 1

85,1 g (1 mol) 2-Pyrrolidon und 2,05 g (30 mmol) Kaliummethanolat wurden vorgelegt und im Vakuum Methanol abdestilliert. 30 g des so erhaltenen Ansatzes wurden bei 150°C und 20 bar Acetylen für 1 h der Vinylierung unterworfen. Die Ausbeute an N-Vinylpyrrolidon (NVP) betrug 69 % der Theorie (gaschromatographisch bestimmt).

### Vergleichsbeispiel 1

85,1 g (1 mol) 2-Pyrrolidon, 2,05 g (30 mmol) Kaliummethanolat und 2,58 g (30 mmol) Gamma-Butyrolacton wurden vorgelegt und im Vakuum Methanol abdestilliert. 30 g des so erhaltenen Ansatzes wurden bei 150°C und 20 bar Acetylen für 1 h der Vinylierung unterworfen. Die Ausbeute an N-Vinylpyrrolidon (NVP) betrug 49 % der Theorie.

### Vergleichsbeispiel 2

85,1 g (1 mol) 2-Pyrrolidon, 2,05 g (30 mmol) Kaliummethanolat und 1,29 g (15 mmol) Gamma-Butyrolacton wurden vorgelegt und im Vakuum Methanol abdestilliert. 30 g des so erhaltenen Ansatzes wurden bei 150°C und 20 bar Acetylen für 1 h der Vinylierung unterworfen. Die Ausbeute an N-Vinylpyrrolidon (NVP) betrug 52 % der Theorie.

### Beispiel 2

85,1 g (1 mol) 2-Pyrrolidon, 2,05 g (30 mmol) Kaliummethanolat und 0,26 g (3 mmol) Gamma-Butyrolacton wurden vorgelegt und im Vakuum Methanol abdestilliert. 30 g des so erhaltenen Ansatzes wurden bei 150°C und 20 bar Acetylen für 1 h der Vinylierung unterworfen. Die Ausbeute an N-Vinylpyrrolidon (NVP) betrug 66 % der Theorie.

## Patentansprüche

1. Verfahren zur Herstellung von N-Vinylpyrrolidon durch Umsetzung von 2-Pyrrolidon mit Acetylen, **dadurch gekennzeichnet, dass** das als Ausgangsstoff eingesetzte 2-Pyrrolidon (im nachfolgenden Ausgangs-2-Pyrrolidon genannt) weniger als 1 Gew. Teile y - Butyrolacton auf 100 Gew. Teile 2- Pyrrolidon enthält.

2. Verfahren gemäß Anspruch 1, **dadurch gekennzeichnet, dass** das Ausgangs-2-Pyrrolidon weniger als 0,15 Gew. Teil y - Butyrolacton enthält.

3. Verfahren gemäß einem der Ansprüche 1 oder 2, **dadurch gekennzeichnet, dass** die Umsetzung des Ausgangs-2-Pyrrolidons mit Acetylen kontinuierlich bei Temperaturen von 120 bis 220°C und Drucken von 1 bis 25 bar erfolgt.

4. Verfahren gemäß einem der Ansprüche 1 bis 3, **dadurch gekennzeichnet, dass** die Umsetzung in Gegenwart von Kaliumpyrrolidat als Katalysator erfolgt.

## Claims

1. A process for preparing N-vinylpyrrolidone by reacting 2-pyrrolidone with acetylene, wherein the 2-pyrrolidone used as a starting material (referred to hereinafter as starting 2-pyrrolidone) comprises less than 1 part by weight of γ-butyrolactone per 100 parts by weight of 2-pyrrolidone.

2. The process according to claim 1, wherein the starting 2-pyrrolidone comprises less than 0.15 part by weight of γ-butyrolactone.

3. The process according to either of claims 1 and 2, wherein the reaction of the starting 2-pyrrolidone with acetylene is effected continuously at temperatures of from 120 to 220°C and pressures of from 1 to 25 bar.

4. The process according to any of claims 1 to 3, wherein the reaction is effected in the presence of potassium pyrrolidate as a catalyst.

## Revendications

1. Procédé pour la préparation de N-vinyl-pyrrrolidone par mise en réaction de 2-pyrrolidone avec de l'acétylène, **caractérisé en ce que** la 2-pyrrolidone utilisée comme produit de départ (dénommée ci-après 2-pyrrolidone de départ) contient moins de 1 partie en poids de γ-butyrolactone pour 100 parties en poids de 2-pyrrolidone.

2. Procédé selon la revendication 1, **caractérisé en ce que** la 2-pyrrolidone de départ contient moins de 0,15 partie en poids de γ-butyrolactone.

3. Procédé selon la revendication 1 ou 2, **caractérisé en ce que** la réaction de la 2-pyrrolidone de départ avec l'acétylène s'effectue en continu à des températures de 120 à 220 °C et sous des pressions de 1 à 25 bars.

4. Procédé selon l'une quelconque des revendications 1 à 3, **caractérisé en ce que** la réaction s'effectue en présence de pyrrolidate de potassium en tant que catalyseur.
